# EUROPEAN PATENT APPLICATION

(11) **EP 2 944 962 A1**
(43) Date of publication of application: **18.11.2015**
(21) Application number: 14168317.7
(22) Date of filing: 14.05.2014
(51) Int. Cl.: G01N 33/68, A61K 47/48

(54) **Method for determining antibody specificity**

(71) Applicant: UCB Biopharma SPRL, 1070 Brussels (BE)
(72) Inventor: The designation of the inventor has not yet been filed
(74) Representative: UCB Intellectual Property

(57) **Abstract**

The present invention relates to a method for determining antibody specificity, whether a multivalent antibody is monospecific, particularly whether a bivalent antibody is monospecific or bispecific. Furthermore the invention provides a method for quantifying the amount of bispecific bivalent antibody present in a sample.

SEQUENCE LISTING

<110>  UCB Biopharma sprl

<120>  Method for determining antibody specificity

<130>  B0089

<160>  5

<170>  PatentIn version 3.5

<210>  1
<211>  327
<212>  PRT
<213>  Artificial Sequence

<220>
<223>  IgG4 heavy chain constant region with S228P mutation

<220>
<221>  MISC_FEATURE
<222>  (108)..(108)
<223>  S228P mutation

<300>
<301>  Angal S1, King DJ, Bodmer MW, Turner A, Lawson AD, Roberts G,
       Pedley B, Adair JR.
<302>  A single amino acid substitution abolishes the heterogeneity of
       chimeric mouse/human (IgG4) antibody.
<303>  Mol Immunol
<304>  30
<305>  1
<306>  105-108
<307>  1993-01

<400>  1

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  2
<211>  447
<212>  PRT

<213>  Homo sapiens

<400>  2

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Phe Asn Asp Tyr
            20                  25                  30

Phe Met Asn Trp Val Arg Gln Ala Pro Gly Lys Gly Leu Glu Trp Val
        35                  40                  45

Ala Gln Met Arg Asn Lys Asn Tyr Gln Tyr Gly Thr Tyr Tyr Ala Glu
    50                  55                  60

Ser Leu Glu Gly Arg Phe Thr Ile Ser Arg Asp Asp Ser Lys Asn Ser
65                  70                  75                  80

Leu Tyr Leu Gln Met Asn Ser Leu Lys Thr Glu Asp Thr Ala Val Tyr
                85                  90                  95

Tyr Cys Ala Arg Glu Ser Tyr Tyr Gly Phe Thr Ser Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Pro Cys Pro Ala Pro Glu Phe Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Arg Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly Lys
        435                 440                 445

<210> 3
<211> 106
<212> PRT
<213> Homo sapiens

<400> 3

Thr Val Ala Ala Pro Ser Val Phe Ile Phe Pro Pro Ser Asp Glu Gln
1               5                   10                  15

Leu Lys Ser Gly Thr Ala Ser Val Val Cys Leu Leu Asn Asn Phe Tyr
            20                  25                  30

Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60

Tyr Ser Leu Ser Ser Thr Leu Thr Leu Ser Lys Ala Asp Tyr Glu Lys
65                  70                  75                  80

His Lys Val Tyr Ala Cys Glu Val Thr His Gln Gly Leu Ser Ser Pro
                85                  90                  95

Val Thr Lys Ser Phe Asn Arg Gly Glu Cys
            100                 105

<210> 4
<211> 330
<212> PRT
<213> Homo sapiens

<400> 4

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  5
<211>  106
<212>  PRT
<213>  Homo sapiens

<400>  5

Thr Val Ala Ala Pro Ser Val Phe Ile Phe Pro Pro Ser Asp Glu Gln
1               5                   10                  15

Leu Lys Ser Gly Thr Ala Ser Val Val Cys Leu Leu Asn Asn Phe Tyr
            20                  25                  30

Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60

Tyr Ser Leu Ser Ser Thr Leu Thr Leu Ser Lys Ala Asp Tyr Glu Lys
65                  70                  75                  80

His Lys Val Tyr Ala Cys Glu Val Thr His Gln Gly Leu Ser Ser Pro
                85                  90                  95

Val Thr Lys Ser Phe Asn Arg Gly Glu Cys
            100                 105

## Description

### FIELD OF THE INVENTION

The invention relates to a method for determining whether a multivalent antibody is monospecific, particularly whether a bivalent antibody is monospecific or bispecific. The invention further relates to a method for quantifying the amount of bispecific bivalent antibody in a sample, and in a more specific manner, the invention relates to a method for quantifying Fab arm exchange of an antibody.

### BACKGROUND OF THE INVENTION

Immunoglobulin G (IgG) is the most abundant antibody (Ab) in human sera and is subdivided into four subclasses: IgG1, IgG2, IgG3, and IgG4. IgG1 and IgG4 both have two interhinge disulphide bonds, IgG2 has four interhinge disulphide bonds, and IgG3 has 11 interhinge disulphide bonds. IgG1 and IgG3 are generally described as active subclasses because they elicit antibody-dependent cell-mediated cytotoxicity and complement-dependent cell-mediated cytotoxicity. Conversely, IgG2 and IgG4 are described as inactive subclasses as they bind poorly to effector molecules, resulting in relatively low effector function induction.

In a therapeutic setting, the choice of antibody has generally been governed by the different properties associated to different antibody subclasses. IgG4 antibodies are generally the subclass of choice where recruitment of immune effector functions is not desired, and the therapeutic effect is achieved for example via receptor blocking or targeted delivery of conjugated effector molecules.

Among the IgG subclasses, IgG4 molecules have a unique feature in that they are able to undergo a dynamic swapping event known as "half-molecule exchange" or "Fab arm exchange" (FAE). This physiological process involves the exchange and recombination of a heavy-light chain pair (half-molecule) of one IgG4 antibody with that of another IgG4 antibody. This effect is due to the presence of an unusual hinge sequence which renders the disulfide bonds between the heavy chains especially susceptible to reduction. Additionally, the IgG4 CH3 domain forms less stable homodimers compared to IgG1 antibodies, also facilitating the exchange (Aalberse et al. 2009).

This inherent ability of bivalent, monospecific IgG4 antibodies to undergo Fab arm exchange results in the formation of bivalent, bispecific antibodies i.e. antibodies which bind with each arm to a different antigen and are thus unable to cross-link identical antigens. Exchange occurring between antibodies with different and unknown variable regions results in bispecific antibodies with unknown, and perhaps undesirable specificity. Furthermore, biotherapeutic bivalent, monospecific IgG4 antibodies undergoing Fab arm exchange with endogenous IgG4 antibodies could result in the formation of exchanged bivalent, but functionally monovalent, bispecific antibodies resulting in a loss of avidity when binding to the target antigen. This could affect the pharmacokinetics and efficacy of the biotherapeutic and change homologous cross-linking of the originally targeted antigen to non-cross-linking behaviour. Subsequently, Fab arm exchange could introduce undesired pharmacodynamic unpredictability for human immunotherapy. Therefore, the propensity of IgG4 antibodies to participate in Fab arm exchange must be understood in order to allow the use of the, otherwise inactive, IgG4 isotype as the backbone for biotherapeutics. There is thus a need in the art to provide assays for determining whether a monovalent antibody is monospecific or not, whether a bivalent antibody is monospecific or bispecific, for quantifying the amount of bispecific bivalent antibody in a sample and to measure (quantify) Fab arm exchange for antibodies, especially for IgG4 antibodies.

In this sense van der Neut Kofschoten et al. 2007 described a sandwich ELISA assay which allowed measurement of Fab arm exchange between an anti-CD20 IgG4 and an anti-EGFR IgG4, using anti-idiotypic antibodies.

In 2011 Shapiro et al. also reported a method for determining Fab arm exchange of an anti-alpha4 IgG4 with endogenous IgG4 based on an ELISA assay.

Despite the above there is a need in the art for further improved methods that provide a reliable method for quantifying Fab arm exchange of antibodies.

### BRIEF DESCRIPTION OF THE FIGURES

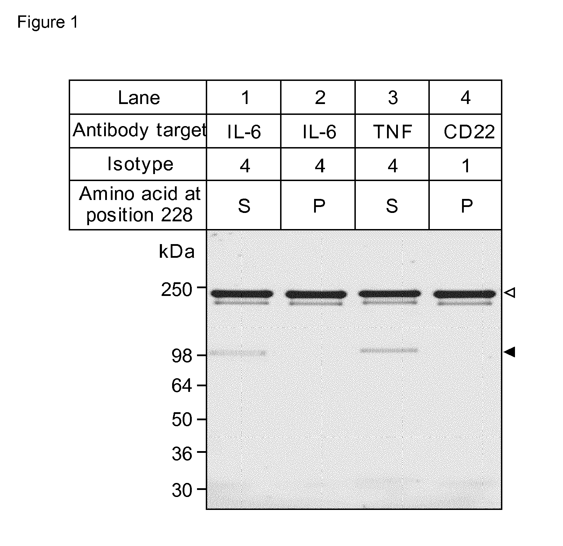

Figure 1: Antibody analysis by SDS-PAGE and Coomassie staining. Equivalent amounts of each antibody were analysed by denaturing, non-reducing SDS-PAGE and Coomassie staining. The isotype, specificity and hinge sequence (wild-type or S228P mutation) of each antibody is specified above. Open arrowhead, indicates the position of intact, full-length Antibodies; Closed arrowhead, indicates the position of half-molecules. Note the absence of detectable half-molecules in the anti-IL-6 clinical candidate S228P IgG4 preparation (lane 2). The positions and molecular masses of protein markers are shown on the left.

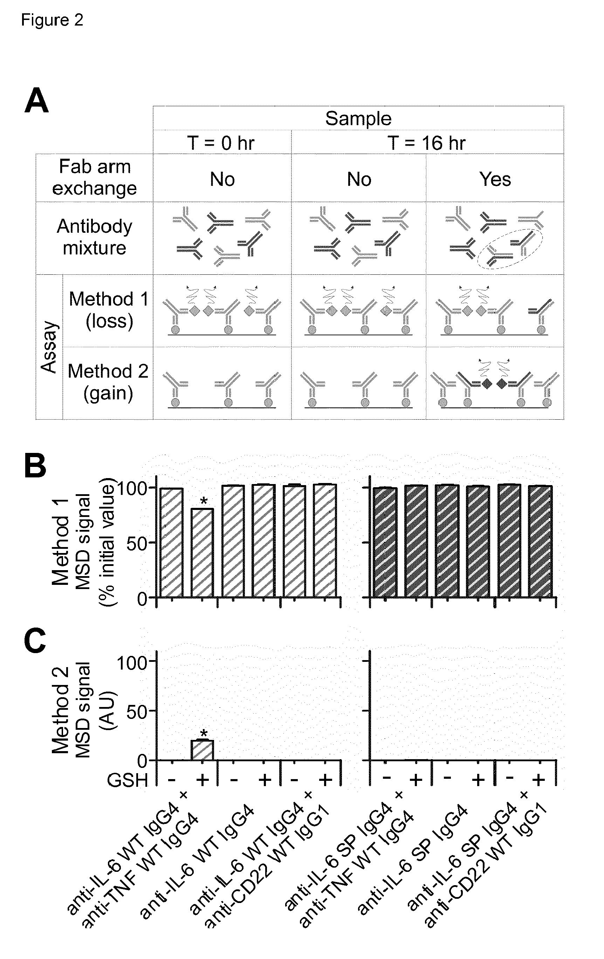

Figure 2: Fab arm exchange. A, schematic representation of *in vitro* FAE and the assayof the invention.: samples are incubated with a mixture of the same antigen molecule bound to one or another of two different detectable markers. A loss in signal after 16 hours incubation infers FAE has taken place (method 1). In parallel, samples are incubated with a mixture of two different antigens each bound to a different detectable marker. A gain in signal after 16 hours incubation infers the presence of newly formed bispecific antibodies arising from FAE (method 2). Light grey Y-shapes, anti-IL-6 (wild-type or S228P) antibodies; dark grey Y-shapes, anti-TNF wild-type antibodies; black broken ellipse, highlights the newly formed bispecific (anti-IL-6/anti-TNF) antibodies; solid black line, streptavidin coated Meso Scale Discovery (MSD) plates; light grey circles, biotinylated IL-6; light grey diamonds, Sulfo-tag™ labelled IL-6; dark grey diamonds, Sulfo-tag™ labelled TNF; curly arrow, denotes signal generated. B and C, quantifying and detecting FAE *in vitro* by two MSD based methods. Antibody samples quenched before or after overnight incubation at 37°C (T=0 and T=16 h, respectively) in the presence or absence of GSH to prevent further FAE, were analysed by the two assays shown in A. Light grey hatched bars, anti-IL-6 wild-type IgG4 antibody containing samples; dark grey hatched bars, anti-IL-6 S228P IgG4 antibody containing samples. Note: Only the sample containing anti-IL-6 wild-type antibodies and anti-TNF wild-type IgG4 antibodies incubated in the presence of GSH shows a loss in signal when assayed by Method 1 (B) and an associated gain in signal when assayed by Method 2 (C). Data points represent mean + SEM values of three independent measurements. *P < 0.05, paired t-test.

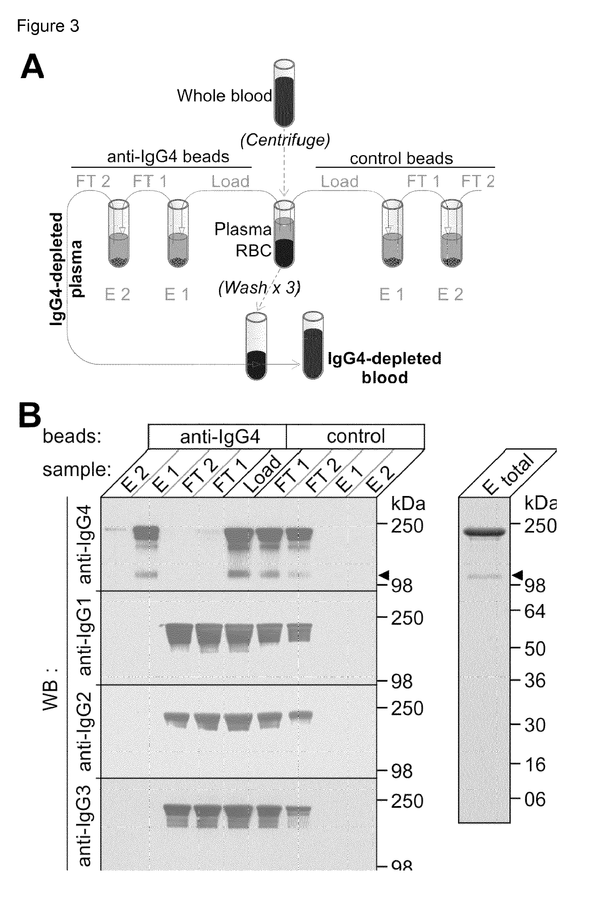

Figure 3: Preparation and analysis of IgG4-depleted physiological matrices. A, schematic of the process using anti-IgG4 (and control) beads employed to deplete endogenous IgG4 antibodies. After centrifuging blood, equivalent amounts of plasma (load) were sequentially loaded onto either, two anti-Ig4 columns (left hand side) or, in parallel, with two control columns (right hand side). The unbound, flow-through (FT) material from each run was collected (FT1 and 2, respectively) before beads were washed and eluted with SDS-PAGE sample buffer (E1 and 2, respectively). FT2 from the anti-IgG4 beads was either used directly (IgG4-depleted plasma) or, was added to washed red blood cells (RBC) before use to yield, IgG4-depleted blood. B, Analysis of resulting samples by SDS-PAGE, WB and Coomassie staining. Samples (from A) were analysed by SDS-PAGE and WB with HRP conjugated anti-IgG 4, 1, 2 and 3 Fc specific antibodies (specified on the left hand side of the main image). Closed arrowhead, denotes the position of half-molecules (only immunoreactive with anti-IgG4 antibodies). Fractions E1 and E2 from incubation with the anti-IgG4 beads were combined (Etotal) and analysed by SDS-PAGE and Coomassie staining (right hand image). The positions and molecular masses of protein markers are shown on the right.

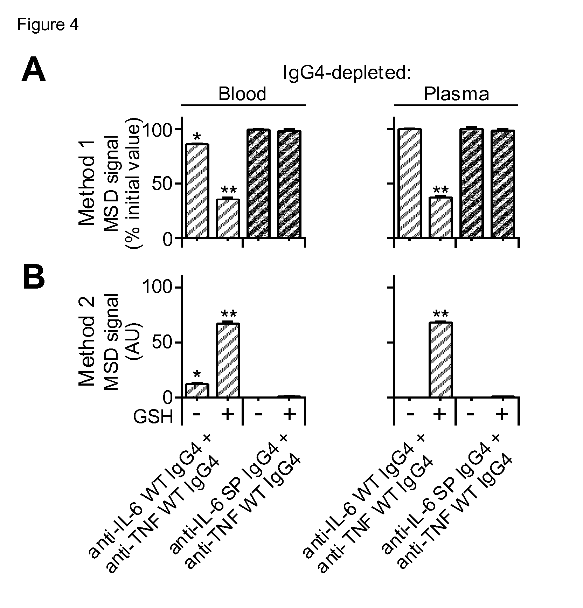

Figure 4: Analysis of FAE in ex vivo IgG-4-depleted physiological matrices. A and B, anti-IgG4 wild-type antibody (light grey hatched bars) or the S228P modified variant thereof (dark grey hatched bars) containing samples were incubated with anti-TNF wild-type IgG4 antibodies at a 1:9 molar ratio in either, IgG4-depleted blood (left hand side) or IgG4-depleted plasma (right hand side), in the presence or absence of GSH. After overnight incubation at 37°C samples were quenched and analysed for FAE by the the method of the invention. A, samples are incubated with a mixture of the same antigen molecule bound to one or another of two different detectable markers (method 1). B, samples are incubated with a mixture of two different antigens each bound to a different detectable marker (method 2). Note: Under physiologically relevant *in vitro* reducing conditions i.e. in the absence of supplemented GSH, FAE only occurs in blood but not plasma. Data points represent mean + SEM values of three independent experiments. *P < 0.05, **P < 0.01, paired t-test.

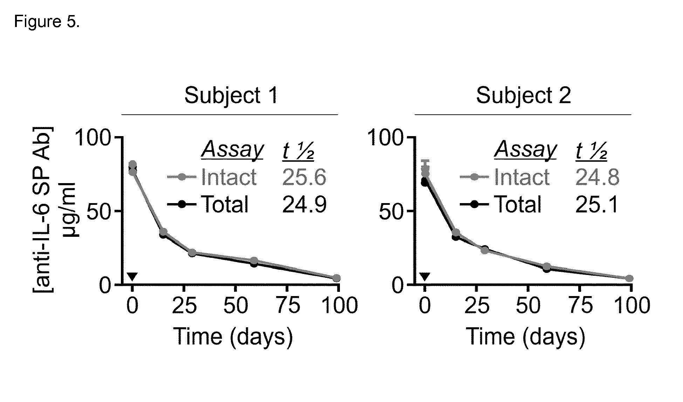

Figure 5: Total and intact S228P anti-IL6 antibody plasma concentration-time profiles of two dosed subjects. Healthy volunteers were given a 3 mg/kg IV dose of S228P anti-IL6 antibody. Plasma samples were taken at regular intervals and assayed by two discrete methods. Total assay; detects all monospecific, monovalent S228P anti-IL6 antibody half-molecules (i.e. both non-exchanged and exchanged species). Intact assay; detects only full-size, monospecific, bivalent S228P anti-IL6 antibody molecules (i.e. only non-exchanged species). Black arrowhead, denotes S228P anti-IL6 antibody administration. Black profile, total assay; grey profile, intact assay; half-lives, (t½).

### DETAILED DESCRIPTION OF THE INVENTION

The present invention addresses the above-identified need by providing a novel method for determining whether a multivalent antibody is monospecific, particularly whether a bivalent antibody is monospecific or bispecific. The invention further provides a novel method for quantifying the amount of bispecific bivalent antibody in a sample, and in a more specific manner, and a method for quantifying Fab arm exchange of an antibody.

In a first embodiment the invention provides an in vitro method for determining whether a sample of bivalent antibody comprises any bispecific antibody having variable region 1 (VR1) and variable region 2 (VR2) comprising:
a) adding an antigen specifically binding to VR1 wherein half of the antigen has a first detectable marker (M1) and the other half has a second detectable marker (M2); and
b) determining whether the sample comprises antibody to which both M1 and M2 are specifically bound;
wherein the sample comprises bispecific antibody if antibody to which M1 and M2 are bound is detected.

In a second embodiment, the invention provides an in vitro method for quantifying the amount of bispecific bivalent antibody, having a variable region 1 (VR1) and a variable region 2 (VR2), in a sample comprising:
a) providing a reference sample wherein the antibody is monospecific having only VR1, and a test sample wherein the amount of bispecific antibody is not known;
b) adding the same antigen specifically binding to VR1 wherein half of the antigen has a first detectable marker (M1) and the other half has a second detectable marker (M2), to each of the samples; and
c) determining the amount of antibody to which both M1 and M2 are specifically bound in each of the samples, and
d) comparing the amount of M1 or M2 in the reference and the test sample.

In a third embodiment, the invention provides an *in vitro* method for quantifying Fab arm exchange of a bivalent monospecific antibody (Ab1) comprising:
a) incubating the antibody (Ab1) with at least a second antibody under conditions such that Fab arm exchange can occur; and
b) quantifying the amount of resulting bivalent antibody according to the method of the second embodiment.

In a fourth embodiment of the invention the bivalent antibody of the first and second embodiments of the invention, or the antibody (Ab1) according to the third embodiment of the invention, is an IgG4. In an alternative embodiment the second antibody according to the third embodiment of the method of the invention is an IgG4. In a further embodiment of the method of the invention according to the third embodiment, both the antibody Ab1 and the second antibody are IgG4. As a skilled artisan would understand, the method according to the third embodiment of the current invention may be used to establish Fab arm exchange of any antibody subclass. This is particularly relevant, in the context of the development of antibodies containing genetically engineered mutations, particularly where these mutations affect the hinge region or CH3-CH3 interface in the antibodies.

In a fifth embodiment of the methods of the invention the determination of the amount of antibody to which both M1 and M2 are bound is performed by allowing M1 to bind to a matrix and measuring the amount of M2 bound to the antibody and captured by the matrix.

In a sixth embodiment of the methods of the invention, M2 is an electrochemi-luminescent marker.

In a seventh embodiment of the invention, the method for quantifying Fab arm exchange according to the third embodiment is characterised in that step a) is performed in blood plasma.

In an eighth embodiment of the invention, the method for quantifying Fab arm exchange is characterized in that said blood plasma is depleted of endogenous IgG4s and wherein the antibody (Ab1) and a second antibody (Ab2) have been added.

As has been previously described in the art, physiological matrices such as blood or plasma provide a naturally reducing environment in which Fab arm exchange occurs. However, in an in vitro situation it is necessary to provide such an environment, typically by the addition of a reducing agent. The skilled artisan is aware of alternative means capable of controlling the redox potential of the sample, thus providing such an environment.

In a ninth embodiment of the invention, said step a) is performed in the presence of a reducing agent selected from among glutathione, mercaptoethanol, dithiothreitol or Tris(2-carboxyethyl)phosphine, and combinations thereof.

Once Fab arm exchange has occurred, the artisan may wish to prevent further Fab arm exchange to take place. As a skilled artisan would know Fab arm exchange between antibodies may be prevented by freezing the sample, for example by storing at -20 °C, or by inactivating the thiol groups present on the antibody.

In a tenth embodiment of the invention, the method for quantifying Fab arm exchange according to the third embodiment of the invention, further comprises preventing Fab arm exchange after step a) by inactivating thiol groups.

In an eleventh embodiment of the invention, said inactivation is performed by adding an alkylating agent.

Examples of alkylating agents known to the skilled artisan include, but are not limited to N-ethylmaleimide (NEM), iodoacetamide (IAM), iodoacetic acid (IAA), acrylamide, 2-vinylpyridine, or 4-vinylpyridine.

In a twelfth embodiment of the invention, said alkylating agent is selected from N-ethylmaleimide, iodoacetamide, iodoacetic acid, and combinations thereof.

In a thirteenth embodiment of the invention, the method for quantifying Fab arm exchange according to the third embodiment of the invention additionally comprises performing another quantification comprising:
a2) incubating the antibody (Ab1) with a second antibody (Ab2) under conditions such that Fab arm exchange can occur;
b2) providing a reference sample wherein the antibody is monospecific having only VR1, and the test sample resulting from a2);
c2) adding a mixture of antigen molecules wherein 50% are antigen 1 (Ag1) specific for Ab1 and bound to M1, and 50% are antigen 2 (Ag2) specific for Ab2 and bound to M2; and
d2) determining the amount of antibody to which both M1 and M2 are specifically bound.

In the fourteenth embodiment of the method for quantifying Fab arm exchange according to the third embodiment of the invention, the increase in M2 signal with respect to its reference sample in c2) correlates with a loss of M2 signal measured in the fifth embodiment of the invention.

As a skilled artisan would understand this correlation between the loss of signal in the first quantification and gain of signal in the second quantification, allows for confirmation of the presence of newly formed bispecific antibodies.

In the fifteenth embodiment of the invention, the reference sample according to method of the third embodiment of the invention, is obtained by performing the method wherein step a) is performed under conditions such that Fab arm exchange can't occur.

For practical purposes from an experimental perspective, measurement from a sample obtained when the reference sample is obtained immediately after contacting both antibodies, is considered time = 0.

In a particular embodiment of the invention, in the method for quantifying Fab arm exchange either of the detectable markers used to measure the assay. are radioactive markers, fluorescent markers, enzyme markers, luminescent, etc. The skilled artisan would know other markers useful for the method of the invention.

In a further particular embodiment of the method for quantifying Fab arm exchange according to the invention, step a) is performed at a temperature from 20°C to 42°C, preferably from 30°C to 38°C, preferably from 35°C to 38°C, and preferably at 37°C.

In a further particular embodiment of the method for quantifying Fab arm exchange according to the invention, the antibody (Ab1) is contacted with at least one other antibody for a determined amount of time. Generally, within physiological ranges the amount of time necessary for such a reaction to occur is inversely proportional to the temperature, and as such the lower the temperature the slower the reaction rate. It is routine for the skilled artisan to establish the best reaction time at a given temperature, and conversely the ideal temperature for a given time-frame. In a particular embodiment, this time is preferably from 2 hours to 48 hours, from 4 hours to 40 hours, from 8 hours to 26 hours, from 10 hours to 24 hours, preferably from 12 hours to 20 hours, and preferably from 16 hours to 18 hours.

In a further particular embodiment of the method for quantifying Fab arm exchange according to the invention the reducing agent is glutathione, preferably it is present at a concentration of from 0.01 mM to 10 mM, preferably from 0.01 mM to 5 mM, preferably from 0.02 mM to 3 mM, preferably from 0.2 mM to 0.8 mM, preferably from 0.3 mM to 0.7 mM, and preferably 0.5 mM. A skilled artisan would routinely establish the necessary equivalent concentrations when using other reducing agents.

In a further particular embodiment of the invention, the detectable marker M1 is bound to the matrix via an antibody specific for M1.

The term "antibody" or "antibodies" as used herein refers to monoclonal or polyclonal antibodies. The term "antibody" or "antibodies" as used herein includes but is not limited to recombinant antibodies that are generated by recombinant technologies as known in the art. "Antibody" or "antibodies" include antibodies' of any species, in particular of mammalian species; such as human antibodies of any isotype, including IgA1, IgA2, IgD, IgG1, IgG2a, IgG2b, IgG3, IgG4 IgE and IgM and modified variants thereof, non-human primate antibodies, e.g. from chimpanzee, baboon, rhesus or cynomolgus monkey; rodent antibodies, e.g. from mouse, rat or rabbit; goat or horse antibodies; and camelid antibodies (e.g. from camels or llamas such as NanobodiesTM) and derivatives thereof; or of bird species such as chicken antibodies or of fish species such as shark antibodies. The term "antibody" or "antibodies" also refers to "chimeric" antibodies in which a first portion of at least one heavy and/or light chain antibody sequence is from a first species and a second portion of the heavy and/or light chain antibody sequence is from a second species. Chimeric antibodies of interest herein include "primatized" antibodies comprising variable domain antigen-binding sequences derived from a non-human primate (e.g. Old World Monkey, such as baboon, rhesus or cynomolgus monkey) and human constant region sequences. "Humanized" antibodies are chimeric antibodies that contain a sequence derived from non-human antibodies. For the most part, humanized antibodies are human antibodies (recipient antibody) in which residues from a hypervariable region of the recipient are replaced by residues from a hypervariable region [or complementarity determining region (CDR)] of a non-human species (donor antibody) such as mouse, rat, rabbit, chicken or non-human primate, having the desired specificity, affinity, and activity. In most instances residues of the human (recipient) antibody outside of the CDR; i.e. in the framework region (FR), are additionally replaced by corresponding non-human residues. Furthermore, humanized antibodies may comprise residues that are not found in the recipient antibody or in the donor antibody. These modifications are made to further refine antibody performance. Humanization reduces the immunogenicity of non-human antibodies in humans, thus facilitating the application of antibodies to the treatment of human disease. Humanized antibodies and several different technologies to generate them are well known in the art. The term "antibody" or "antibodies" also refers to human antibodies, which can be generated as an alternative to humanization. For example, it is possible to produce transgenic animals (e.g., mice) that are capable, upon immunization, of producing a full repertoire of human antibodies in the absence of production of endogenous murine antibodies. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies with specificity against a particular antigen upon immunization of the transgenic animal carrying the human germ-line immunoglobulin genes with said antigen. Technologies for producing such transgenic animals and technologies for isolating and producing the human antibodies from such transgenic animals are known in the art. Alternatively, in the transgenic animal; e.g. mouse, only the immunoglobulin genes coding for the variable regions of the mouse antibody are replaced with corresponding human variable immunoglobulin gene sequences. The mouse germline immunoglobulin genes coding for the antibody constant regions remain unchanged. In this way, the antibody effector functions in the immune system of the transgenic mouse and consequently the B cell development is essentially unchanged, which may lead to an improved antibody response upon antigenic challenge *in vivo.* Once the genes coding for a particular antibody of interest have been isolated from such transgenic animals the genes coding for the constant regions can be replaced with human constant region genes in order to obtain a fully human antibody. Other methods for obtaining human antibodies/antibody fragments *in vitro* are based on display technologies such as phage display or ribosome display technology, wherein recombinant DNA libraries are used that are either generated at least in part artificially or from immunoglobulin variable (V) domain gene repertoires of donors. Phage and ribosome display technologies for generating human antibodies are well known in the art. Human antibodies may also be generated from isolated human B cells that are ex vivo immunized with an antigen of interest and subsequently fused to generate hybridomas which can then be screened for the optimal human antibody. The term "antibody" or "antibodies" as used herein, also refers to an aglycosylated antibody.

In certain embodiments of this invention, the antibodies are antibodies that are modified by covalent attachment of functional moieties such as water-soluble polymers, such as poly(ethyleneglycol), copolymers of poly(ethyleneglycol) and poly(propyleneglycol), carboxymethyl cellulose, dextran, poly(vinylalcohol), poly(vinylpyrrolidone) or poly(proline) -- all of which are known to exhibit substantially longer half-lives in blood following intravenous injection than do the corresponding unmodified proteins.

In some embodiments, antibodies of the present invention are antibodies attached to functional moieties such as to poly(ethyleneglycol) (PEG) moieties. In one particular embodiment, the antibody is an antibody fragment and the PEG molecules may be attached through any available amino acid side-chain or terminal amino acid functional group located in the antibody fragment, for example any free amino, imino, thiol, hydroxyl or carboxyl group. Such amino acids may occur naturally in the antibody fragment or may be engineered into the fragment using recombinant DNA methods (see for example US 5,219,996; US 5,667,425; WO 98/25971).

The term "antibody" or "antibodies" as used herein not only refers to untruncated antibodies of any species, including from human (e.g. IgG) and other mammalian species, but also refers to an antibody fragment. A fragment of an antibody comprises at least one heavy or light chain immunoglobulin domain as known in the art and binds to one or more antigen(s). Examples of antibody fragments according to the inventon include Fab, Fab', F(ab')₂, and Fv and scFv fragments; as well as diabodies, triabodies, tetrabodies, minibodies, domain antibodies, single-chain antibodies, bispecific, trispecific, tetraspecific or multispecific antibodies formed from antibody fragments or antibodies, including but not limited to Fab-Fv constructs. Antibody fragments as defined above are known in the art.

The term "bivalent antibody" as used herein means an antibody with 2 antigen-binding sites.

The term "monospecific antibody" as used herein means an antibody that contains identical variable regions.

The term "bispecific antibody" as used herein means an antibody that contains two different variable regions.

The term "Fab arm exchange" or "FAE" as used herein means the exchange and recombination of one complete heavy-light chain pair (half-molecule) of one antibody with that of another antibody. IgG4 antibodies are most likely to undergo Fab arm exchange.

The term "conditions such that Fab arm exchange can occur" as used herein means conditions wherein the exchange and recombination of a heavy-light chain pair (half-molecule) of one antibody with that of another antibody are able to take place. Typically, Fab arm exchange occurs in the presence of a reducing environment. As a skilled artisan would know said reducing environment is naturally present in the bloodstream *in vivo,* and therefore also in blood and serum samples obtained from a donor. However, a reducing environment can be recreated in *in vitro* situations by adding a reducing agent, such as defined below and disclosed throughout this text.

Conversely "conditions such that Fab arm exchange can't occur" as used herein means conditions wherein the exchange and recombination of a heavy-light chain pair (half-molecule) of one antibody with that of another antibody aren't able to take place. As a skilled artisan would know, this is generally achieved by freezing the sample so that the molecules cannot react, or by inactivating free thiols present on the antibody. Inactivation of free thiols is typically achieved by incubating the antibody with an alkylating agent such as defined above and disclosed throughout this text.

"Preventing Fab arm exchange" means creating conditions in which said exchange is substantially reduced, preferably reduced by 90%, reduced by 95%, or more preferably reduced by 99%. Typically this is achieved by freezing the sample, by inactivating reactive thiol groups, or by combining both. A skilled artisan would understand alternative means of preventing said Fab arm exchange, such as for example by the use of non-reducing conditions.

The term "alkylating agent" as used herein means a substance that causes replacement of hydrogen by an alkyl group. Examples of alkylating agents within the meaning of the present invention include but are not limited to N-ethylmaleimide (NEM), iodoacetamide, and iodoacetic acid, other alkylating reagents also include acrylamide, 2-vinylpyridine, 4-vinylpyridine.

As used herein the term "binding", "specifically binding", "specifically bound" in the context of the binding of an antibody to a predetermined antigen means a binding with an affinity corresponding to a K_{D} of about 10⁻⁷ M or less, such as about 10⁻⁸ M or less, such as about 10⁻⁹ M or less, about 10⁻¹⁰ M or less, or about 10⁻¹¹ M or even less when determined for instance by surface plasmon resonance (SPR) technology in a BIAcore 3000 instrument using the antibody as the analyte. This term also means that the antibody binds to the predetermined antigen with an affinity corresponding to a K_{D} that is at least ten-fold lower, such as at least 100 fold lower, for instance at least 1000 fold lower, such as at least 10,000 fold lower, for instance at least 100,000 fold lower than its affinity for binding to a non-specific antigen (e;g; bovine serum albumin, casein) other than the predetermined antigen or a closely-related antigen. The amount with which the affinity is lower is dependent on the K_{D} of the antibody, so that when the K_{D} of the antibody is very low (that is, the antibody is very specific), then the amount with which the affinity for the antigen is lower than the affinity for a non-specific antigen may be at least 10,000 fold.

The term "K_{D}" as used herein, means the dissociation rate constant of a particular antibody-antigen interaction.

The term "antigen" as used herein means a molecule capable of binding specifically to a particular antibody molecule in the sense defined above.

The term "marker" or "detectable marker" as used herein means a structure that can be specifically detected. Markers are typically molecules that can be chemically linked or conjugated to a protein of interest or other molecule. As known to a person skilled in the art, there are many different markers that may be used in a method of the present invention. Non-limiting examples of markers include enzymes, such as horseradish peroxidase (HRP), alkaline phosphatase (AP) or glucose oxidase. These enzymes allow for detection often because they produce an observable color change in the presence of certain reagents. In some cases these enzymes are exposed to reagents which cause them to produce light. Luminescence is described as the emission of light from a substance as it returns from an electronically excited state to ground state. The various forms of luminescence (bioluminescence, chemiluminescence, photoluminescence) differ in the way the excited state is reached. For example, photoluminescence is simply fluorescence; the excitation is initiated by light at a particular wavelength. Bioluminescence is characterized by the use of a bioluminescent compound, such as luciferin and firefly luciferase. Chemiluminescence is light produced by a chemical reaction whereas electrochemiluminescence is produced in response to an electrical current. Non-limiting examples of electrochemiluminescent markers include ruthenium and osmium polypyridine complex systems. Further examples of markers include radioactive isotopes wherein radioactivity emitted can be easily detected using conventional methods. DNA reporters may also be used as markers, and also fluorogenic reporters such as but not limited to phycoerythrin.

The term "matrix" as used herein means a physical support on which a binding molecule can be attached. As a skilled artisan would know within the present invention, different forms of matrix may be chosen, such as for example a solid matrix. Solid matrices commonly used in the art include beads (e.g. agarose, sepharose, latex, magnetic, , nanoparticles), multi-well plates (e.g. polystyrene, glass), membranes (e.g. nitrocellulose, PVDF).

The term "reducing agent", "reductant" or "reducer" as used herein means an element or compound that loses (or donates) an electron to another chemical species in a redox chemical reaction. Since the reducing agent is losing electrons, it is said to have been oxidized. Within the meaning of the present invention, as a skilled artisan would understand, the reducing agent allows di-sulphide groups to become reactive by generating thiol (-SH) groups. Non-limiting examples of reducing agent include glutathione, mercaptoethanol (b-ME), dithiothreitol (DTT) or Tris(2-carboxyethyl)phosphine (TCEP).

"Inactivating reactive thiol groups" as used herein means blocking all the free thiol groups present in a protein of interest or other molecule to prevent unwanted thiol-disulphide exchange reactions. This is typically achieved using an alkylating agent.

### EXAMPLES

### Materials and methods

### Antibodies and reagents.

Humanised anti-IL-6 wild-type IgG4 antibody, its S228P point mutated variant anti-IL-6 S228P IgG4 antibody, humanised anti-TNF wild-type IgG4 antibody and humanised anti-CD22 IgG1 wild-type antibody were expressed and purified as described in Peters et al. 2012. See SEQ ID NO: 1 for the wild-type constant region of IgG4, and SEQ ID NO: 2 for the mutated constant region (in this sequence the Serine to Proline mutation appears at position 108, given that the original reference was based on a full length antibody sequence). Additionally, SEQ ID NO: 3 corresponds to the constant region of the IgG4 light chain, whereas SEQ ID NO: 4 and 5 correspond to IgG1 constant region heavy and light chains, respectively

Recombinant IL-6, TNF (Peprotech) and anti-human κ-light chain specific antibody (Jackson ImmunoResearch Laboratories) were either biotinylated or Sulfo-tag™ labelled using Sulfo-NHS-LC-LC-Biotin (Thermo Scientific) or ruthenium-NHS-ester (Meso Scale Discovery), respectively, according to the manufacturer's protocols.

### Patient samples - in vivo Fab arm exchange.

Anonymised plasma samples from healthy volunteers who received a single 3 mg/kg dose of S228P anti-IL6 antibody by intravenous injection were obtained from a Phase 1, randomised, double-blind, placebo-controlled study.

### Fab arm exchange

Anti-IL-6 wild-type IgG4 or anti-IL-6 S228P IgG4 antibodies and their potential exchange partners, namely, either anti-TNF wild-type IgG4 or anti-CD22 wild-type IgG1 antibodies, were mixed as follows:
For *in vitro* studies: at 1:1 molar ratios at a total concentration of 100 µg/ml in phosphate-buffered saline (PBS) at pH 7.4.
For ex *vivo* studies: at 1:9 molar ratios at a total concentration of 600 µg/ml in either IgG4-depleted plasma or IgG4-depleted blood (see results).

To allow disulphide bond reduction, samples were supplemented with reduced glutathione (GSH) (Sigma) to a final concentration of 0.5 mM. At the start of the experiment (t=0 h) an aliquot of the mixture received N-ethylmaleimide (NEM) (Sigma) to a final concentration of 10 mM (to inactivate potentially reactive thiol groups, thus inhibiting Fab arm exchange) and was incubated alongside the rest of the reaction for 16 hours at 37°C (t=16 h). After overnight incubation, the t=16 h thiol groups in the sample were also inactivated as above.

### Non-reducing SDS-PAGE, Coomassie staining and Western blotting.

Antibody and samples obtained from the IgG4 depletion process were incubated at 100 °C for 3 minutes in 1X sodium-dodecyl-sulphate polyacrylamide gel electrophoresis (SDS-PAGE) sample buffer supplemented with NEM (to a final concentration of 10 mM) to minimize artifacts and then analysed using 4-20 % gradient Tris-Glycine gels (Invitrogen). Following SDS-PAGE, gels were either stained with Coomassie or transferred to nitrocellulose membrane for Western blotting. Membranes were blocked in 5 % milk (w/v) in PBS/0.1 % Tween-20 (PBST, blocking buffer) for 1 hour at 20°C with agitation before incubation with appropriate horseradish peroxidase (HRP)-conjugated primary antibodies (AbD Serotec 1:2,000 in blocking buffer). The membranes were then washed with PBST, incubated with chemiluminescent substrate and imaged using the ImageQuant 4000 LAS analyser (GE Healthcare). Total protein concentration was quantified by measuring absorbance at 280 nm.

### IgG4 depletion and physiological matrix preparation.

Whole blood from healthy human donors was collected in heparin-containing vials (BD Bioscience) and centrifuged at 1,500 g for 10 minutes at 20°C to separate plasma from cells. 1.5 ml of plasma was sequentially incubated with 2 x 250 µl of Capture Select IgG4 beads (BAC Netherlands) for 1 hour with agitation at 20°C. Following incubation, the unbound flow-through material (IgG4-depleted plasma) was collected and stored at 4°C. The beads were washed 3 times with 1 ml of physiological salt solution (PSS, in mM: 145 NaCl, 5.6 KCI, 5.6 glucose, 1 MgCl₂, 1 CaCl₂, 15 HEPES; pH 7.4) before elution with 100 µl of 1X SDS-sample buffer (Invitrogen). Resulting fractions were then analysed by non-reducing SDS-PAGE (see below).

During IgG4 depletion from plasma, the pelleted blood cells from the initial whole blood centrifugation were washed 3 times by resuspension in PSS and centrifugation as above. The washed blood cells were then resuspended in 'IgG4-depleted plasma' (i.e. the flow-through from the Capture Select IgG4 beads) to yield 'IgG4-depleted whole blood'.

### Detection and quantification of Fab arm exchange (FAE) in vitro.

*In vitro* Fab arm exchange of anti-IL-6 (wild-type or S228P) antibodies with anti-TNF wild-type antibodies was quantified, and the presence of newly formed anti-IL-6/anti-TNF bispecific antibodies (bispecific antibodies) demonstrated, using the method of the invention. In this case the method of the invention was implemented using MSD (Meso Scale Discovery) multi-array plates as a matrix. These plates include high capacity carbon electrodes in each well, which allow for measurement of an electro-chemiluminescent signal in a Sector Imager 6000 analyser (Meso Scale Discovery).

Firstly an indirect quantification of FAE was determined by the 'loss' in ability of pre-existing, parental, bivalent, monospecific anti-IL-6 antibodies to cross-link two differently tagged variants of the same antigen. Reaction samples recovered after incubation with NEM (to prevent further FAE) were diluted 1:4,000 in PBS/1 % bovine serum albumin (PB) and incubated with 2 µg/ml of a 1:1 mixture of biotinylated IL-6 and Sulfo-tag labelled IL-6 for 1 hour at 20°C with agitation. After incubation, samples were transferred to, and incubated with agitation in, PB pre-blocked streptavidin coated MSD plates for 1 h at 20°C before being processed as described below.

Secondly FAE was directly monitored as determined by the 'gain' in ability of the newly formed anti-IL-6/anti-TNF bispecific antibodies to cross-link two different antigens. 1:4,000 PB diluted reaction samples were incubated with 2 µg/ml of a 1:1 mixture of biotinylated-IL-6 and Sulfo-tag™ labelled-TNF in PB for 1 hour at 20°C with agitation. After incubation, samples were transferred to, and incubated with agitation in, PB pre-blocked streptavidin coated MSD plates for 1 hour at 20°C.

After incubation with tagged antigens, wells were washed three times with PBST before signals were revealed using the manufacture's read buffer and measured in a Sectorlmager 6000 analyser (Meso Scale Discovery). Background values obtained from control parallel reactions in which non-biotinylated-IL-6 was substituted for the biotinylated variant, were subtracted from all signals. Duplicate values from at least 3 independent experiments were used for all calculations. Averaged data with standard error of the mean (SEM) values are plotted. Average data was calculated by adding together the MSD values and dividing by the number of replicate readings. Standard error of the mean (SEM) was calculated by dividing the standard deviation of the readings by the square root of the number of independent experiments.

### Detection of S228P anti-IL6 IgG4 antibody Fab arm exchange in vivo.

Plasma samples from healthy volunteers dosed with S228P anti-IL6 IgG4 antibody were taken at regular time intervals and assayed by two different MSD assays namely, total and intact. The total assay detects all monovalent antibody half-molecules (i.e. both non-exchanged and exchanged species) while the intact assay only detects monospecific, bivalent antibody molecules (i.e. only non-exchanged species).

In brief, for the total assay, plasma samples were serially diluted in PB and incubated with agitation for 1 hour at 20°C with 1 µg/ml of biotinylated IL-6. After incubation, samples were transferred to, and incubated with agitation in PB pre-blocked streptavidin coated MSD plates for 1 h at 20°C. Wells were then washed three times with PBST before incubation with 1 µg/ml of Sulfo-tag™ labelled goat anti-human κ-light chain antibody (to label captured S228P anti-IL6 IgG4 antibody κ-light chain half-molecules) in PB for another 1 hour at 20°C with agitation and processed as per the intact assay below.

For the intact assay, after dilution with PB, plasma samples were incubated with agitation for 1 hour at 20°C with 2 µg/ml of a 1:1 mixture of biotinylated IL-6 and Sulfo-tag™ labelled IL-6. After incubation, wells were washed with PBST and signals revealed and measured as previously detailed.

Known amounts of serially diluted S228P anti-IL6 IgG4 antibody were used in parallel assays to obtain calibration curves from which the plasma S228P anti-IL6 IgG4 antibody concentrations were calculated. Duplicate values from 2 independent experiments were used for all calculations. The amount of S228P anti-IL6 IgG4 antibody FAE was assessed by comparing the two generated profiles. *In vivo* plasma S228P anti-IL6 IgG4 half-lives were calculated using GraphPad Prism software.

### Results

### Analysis of antibodies by non-reducing SDS-PAGE

To investigate FAE four different antibodies were used: anti-IL-6 wild-type IgG4, anti-IL-6 S228P IgG4, anti-TNF wild-type IgG4 and anti-CD22 wild-type IgG1. Their analysis by denaturing, non-reducing SDS-PAGE and Coomassie staining is shown in Figure 1. As can be seen from the gel, the wild-type IgG4 antibodies are a heterogenous preparation containing two predominant species: the full-size, bivalent, monospecific antibody (Figure 1 open arrowhead) and a lower molecular weight species corresponding to the monovalent, monospecific, heavy-light chain half-molecules (Figure 1 closed arrowhead). As previously described in Angal et al. 1993, this IgG4 wild-type specific heterogeneity is abolished by the introduction of a single S228P point mutation, numbered according to the EU numbering system (Kabat et al. 1991). Figure 1. Note the absence of heavy-light chain bands in lane 2 (S228P IgG4) and lane 4 (wild-type IgG1).

### Investigating Fab arm exchange in vitro

In order to study the effect of the S228P mutation on anti-IL-6 IgG4 FAE *in vitro,* anti-IL-6 wild-type IgG4 or anti-IL-6 S228P IgG4 antibodies were mixed with either anti-TNF wild-type IgG4 or anti-CD22 wild-type IgG1 antibodies in a 1:1 molar ratio and the samples were incubated at 37°C in the presence or absence of the reducing agent, glutathione (GSH). To control for the potential detrimental effect of GSH, we also incubated anti-IL-6 wild-type IgG4 or anti-IL-6 S228P IgG4 on their own in the presence of GSH in a parallel set of reactions. After incubation, the samples were analysed for FAE by the method of the invention (Figure 2A). Firstly, an MSD based method was used that exploits the bivalent, monospecific nature of the parental antibodies and involves incubation of the reaction mixture with differently tagged variants of the same antigen. A loss in MSD signal by this method infers the loss of parental IgG4 monospecific, bivalent antibodies from the reaction mixture - possibly due to FAE with a counterpart antibody (method 1). Analysis of reaction mixtures by this method resulted in a significant loss (∼ 28 %) in signal from samples containing both anti-IL-6 wild-type IgG4 antibody and anti-TNF wild-type IgG4 antibody supplemented with GSH (Figure 2B). Interestingly, no such loss was observed in the absence of GSH, the anti-IL-6 wild-type IgG4 alone, nor when incubated with wild-type IgG1, or any of the S228P IgG4 containing samples.

Taken together these results infer that anti-IL-6 wild-type monospecific, bivalent IgG4 antibodies are specifically 'lost' from the reaction mixture due to GSH dependent FAE with wild-type anti-TNF IgG4 (but not wild-type IgG1) antibodies. If true, this process ought to result in the formation of anti-IL-6/anti-TNF bispecific antibodies.

In order to directly investigate the presence of anti-IL-6/anti-TNF bispecific antibodies, equivalent aliquots of all reaction mixtures were assayed using a second MSD based method (see Figure 2A). This method exploits the bivalent, bispecific nature of the newly formed antibodies and involves incubation of the reaction mixture with a combination of antigens to first capture and then reveal the bispecific antibodies. A gain in MSD signal by this method infers the formation of newly formed bispecific antibodies from the FAE of disparate, monospecific parental antibodies (method 2. The results of this analysis, Fig. 2C, show an associated complementary, specific and significant, gain in signal in the same reactions which previously showed a loss in signal by Method 1 (namely, the GSH supplemented, anti-IL-6 wild-type IgG4 antibody and anti-TNF wild-type IgG4 antibody sample). No such gain in signal was detected in the absence of GSH, the anti-IL-6 wild-type IgG4 alone, or any of the S228P IgG4 containing samples.

Taken together these results demonstrate that, under these conditions, the wild-type IgG4 antibodies 'lost' from solution (as observed in Figure 2B Method 1) have participated in GSH-dependent FAE with wild-type IgG4 (but not wild-type IgG1) antibodies resulting in the formation of anti-IL-6/anti-TNF bispecifc antibodies (as observed in Figure 2C Method 2).

### Depleting plasma and blood of endogenous IgG4 antibodies.

While PBS provides an antibody-free and buffered environment to monitor Fab arm exchange, the lack of cells and endogenous factors means this *in vitro* buffer is far from physiologically relevant. Much more appropriate physiological media to monitor FAE would be blood or plasma. However, the disadvantage of these *ex vivo* matrices is the presence and abundance of endogenous IgG4 wild-type antibodies of unknown specificity which, due to their intrinsic ability to participate in FAE would result in dilution of the formation of our bispecific antibodies of interest, (namely, anti-IL-6/anti-TNF). This would ultimately result in the underestimation of FAE. Therefore, in order to accurately monitor *in vitro* FAE of the antibodies of interest under physiologically relevant conditions, a novel, IgG4-free, buffered medium was established by depleting blood of endogenous IgG4 antibodies using anti-IgG4 beads (Capture Select IgG4, BAC Netherlands), Figure 3A. After centrifuging blood the top plasma layer was harvested and, to ensure exhaustive IgG4 depletion, subjected to two rounds of anti-IgG4 beads. To control for non-specific absorption of the IgG4 antibodies to the affinity matrix a control beads were used in parallel. Resulting samples were analysed by non-reducing SDS-PAGE and Western blot using HRP-conjugated anti-IgG4 antibodies (Fig. 3B, left upper panel). To verify and confirm that only IgG4 antibodies were being isolated during this process samples were additionally analysed by SDS-PAGE and Western blot using anti-IgG 1, 2 and 3 HRP conjugated antibodies (Figure 3B, left lower panels). The fractions containing IgG4 antibodies were pooled and the purity (> 99 %) assessed by SDS-PAGE and Coomassie staining (Figure 3B, right). By measuring absorbance at 280 nm the total protein content of the pooled anti-IgG4 eluates was determined to be 600 µg/ml. The unbound material recovered after incubation with the anti-IgG4 beads yielded 'IgG-4 depleted plasma' and after addition of washed blood cells yielded 'IgG4 depleted whole blood' (Figure 3A). These two physiologically relevant, buffered, IgG4-free matrices were subsequently used to monitor FAE.

### In vitro S228P anti-IL6 IgG4Fab-arm exchange in IgG4-depleted physiological matrices.

To ensure that physiologically relevant antibody concentrations were experimentally employed for studying FAE, the 600 µg/ml of endogenous IgG4 antibodies removed from whole plasma using anti-IgG4 beads (see above) were replaced with an equivalent amount of pre-mixed anti-IL-6 (either wild-type or S228P) IgG4 antibodies and anti-TNF wild-type IgG4 antibodies. To ensure clinically relevant amounts of antibodies were used, 60 µg/ml of anti-IL-6 IgG4 antibodies (∼ equivalent to the Cmax of a 3 mg/kg IV dose, J. Jose personal communication) were mixed with 540 µg/ml (i.e. a 9-fold molar excess) of anti-TNF wild-type IgG4 antibodies. The antibodies were incubated in either of the two IgG4-depleted matrices in the absence or presence of GSH at 37°C for 16 hours before analysis of FAE by the previously described MSD based methods.

Analysis of reaction mixtures by the method of the invention demonstrated a significant loss in MSD signal (∼ 66 % in IgG4-depleted blood and ∼ 63 % in IgG4-depleted plasma) of the wild-type (but not S228P) anti-IL-6 IgG4 antibodies incubated with anti-TNF wild-type IgG4 antibodies during GSH-dependent incubation at 37°C (Figure 4A, method 1).

Additional analysis of these same samples by a further MSD-based method using an incubation with a combination of IL-6 and TNF as antigens showed a specific and significant gain in MSD signal in the same reactions which had previously demonstrated a loss in signal (Figure 4B). Taken together, these results suggest that the anti-IL-6 wild-type but not S228P IgG4 antibodies undergo FAE with anti-TNF wild-type IgG4 antibodies in the presence of GSH under these ex *vivo* conditions.

Interestingly, even in the absence of supplemented GSH, the wild-type antibody variant (but not the S228P mutant) also participates, to a significant and detectable level, in FAE in IgG4-depleted whole blood (but not in IgG4-depleted plasma) (Figure 4). Since the only difference between these two matrices is the presence of blood cells, these data suggest that it is these, or a component thereof, which is catalysing the FAE reaction.

### In vivo S228P anti-IL6 IgG4 Fab arm exchange.

To date, the propensity of S228P anti-IL6 IgG4 to participate in *in vitro* FAE by mimicking physiologically and clinically relevant *in vivo* conditions has been investigated. However, in order to directly investigate *in vivo* S228P anti-IL6 IgG4 FAE, plasma samples taken from anonymised human volunteers of an S228P anti-IL6 IgG4 clinical trial by two MSD assays (as detailed in materials and methods) were assayed. The first MSD assay (total) was designed to measure both exchanged and non-exchanged monovalent S228P anti-IL6 IgG4 half-molecules able to bind IL-6 antigen while the second assay (intact) was designed to measure only non-exchanged, monospecific, bivalent S228P anti-IL6 IgG4 molecules. Comparison of the plasma concentration profiles from these two methods would be indicative of the extent of *in vivo* S228P anti-IL6 IgG4 FAE. However, as can be seen from Figure 5, analysis of S228P anti-IL6 IgG4 by the two methods produced overlapping S228P anti-IL6 IgG4 plasma concentration profiles for both clinical subjects tested. This infers that S228P anti-IL6 IgG4 does not participate, to any significant level, in *in vivo* Fab arm exchange.

### CITED REFERENCES

Aalberse, R.C., et al., Immunoglobulin G4: an odd antibody. Clin Exp Allergy, 2009. 39(4): p. 469-77.
Angal, S., et al., A single amino acid substitution abolishes the heterogeneity of chimeric mouse/human (IgG4) antibody. Mol Immunol, 1993. 30(1): p. 105-8.
C. Jacob, et al. Sulfur and selenium: the role of oxidation state in protein structure and function. Chem., Int. Ed., 2003, 42, 4742-4758).
Kabat, E.A., et al., Sequences of Proteins of Immunological Interest. Fifth edition ed1991.
Peters, S.J., et al., Engineering an improved IgG4 molecule with reduced disulfide bond heterogeneity and increased Fab domain thermal stability. J Biol Chem, 2012. 287(29): p. 24525-33.
Shapiro, R.I., et al., Development and validation of immunoassays to quantify the half-antibody exchange of an IgG4 antibody, natalizumab (Tysabri(R)) with endogenous IgG4. J Pharm Biomed Anal, 2011. 55(1): p. 168-75.
van der Neut Kolfschoten, M., et al., Anti-inflammatory activity of human IgG4 antibodies by dynamic Fab arm exchange. Science, 2007. 317(5844): p. 1554-7.

## Claims

1. An in vitro method for determining whether a sample of bivalent antibody comprises any bispecific antibody having variable region 1 (VR1) and variable region 2 (VR2) comprising:
a) adding an antigen specifically binding to VR1 wherein half of the antigen has a first detectable marker (M1) and the other half has a second detectable marker (M2); and
b) determining whether the sample comprises antibody to which both M1 and M2 are specifically bound;
wherein the sample comprises bispecific antibody if antibody to which M1 and M2 are bound is detected.

2. An in vitro method for quantifying the amount of bispecific bivalent antibody, having a variable region 1 (VR1) and a variable region 2 (VR2), in a sample comprising:
a) providing a reference sample wherein the antibody is monospecific having only VR1, and a test sample wherein the amount of bispecific antibody is not known;
b) adding an antigen specifically binding to VR1 wherein half of the antigen has a first detectable marker (M1) and the other half has a second detectable marker (M2), to each of the samples; and
c) determining the amount of antibody to which both M1 and M2 are specifically bound in each of the samples, and
d) comparing the amount of M1 or M2 in the reference and the test sample.

3. An *in vitro* method for quantifying Fab arm exchange of a bivalent monospecific antibody (Ab1) comprising:
a) incubating the antibody (Ab1) with at least a second antibody under conditions such that Fab arm exchange can occur; and
b) quantifying the amount of resulting bivalent antibody according to the method of claim 2.

4. A method according to any preceding claim wherein the bivalent antibody to be analysed is an IgG4.

5. A method according to any preceding claim wherein the determination of the amount of antibody to which both M1 and M2 are bound is performed by allowing M1 to bind to a matrix and measuring the amount of M2 bound to the antibody and captured by the matrix.

6. A method according to claim 5 wherein M2 is an electrochemi-luminescent marker.

7. A method according to claim 3 wherein step a) is performed in blood plasma.

8. A method according to claim 7 wherein the blood plasma is depleted of endogenous IgG4s and wherein the antibody (Ab1) and a second antibody have been added.

9. A method according to claim 3 wherein step a) is performed in the presence of a reducing agent selected from among glutathione, mercaptoethanol, dithiothreitol or Tris(2-carboxyethyl)phosphine, and combinations thereof.

10. A method according to any of claims 3 to 9, comprising preventing Fab arm exchange after step a) by inactivating thiol groups.

11. A method according to claim 10 wherein said inactivation is performed by adding an alkylating agent.

12. A method according to claim 11 wherein said alkylating agent is selected from N-ethylmaleimide, iodoacetamide, iodoacetic acid, and combinations thereof.

13. A method according to any of claims 3 to 12, additionally comprising performing another quantification comprising:
a2) incubating the antibody (Ab1) with a second antibody (Ab2) under conditions such that Fab arm exchange can occur;
b2) providing a reference sample wherein the antibody is monospecific having only VR1, and the test sample resulting from a2);
c2) adding a mixture of antigen molecules wherein 50% are antigen 1 (Ag1) specific for Ab1 and bound to M1, and 50% are antigen 2 (Ag2) specific for Ab2 and bound to M2; and
d2) determining the amount of antibody to which both M1 and M2 are specifically bound.

14. A method according to claim 13 wherein the increase in M2 signal with respect to its reference sample in c2) correlates with a loss of M2 signal measured in claim 5.

15. A method according to any of claims 3 to 13, wherein the reference sample is obtained by performing the method wherein step a) is performed under conditions such that Fab arm exchange can't occur.
